# EUROPÄISCHE PATENTANMELDUNG

(11) **EP 1 953 128 A1**
(43) Veröffentlichungstag der Anmeldung: **06.08.2008**
(21) Anmeldenummer: 08000689.3
(22) Anmeldetag: 15.01.2008
(51) Int. Cl.: C07C 4/12, C10G 35/24

(54) **Verfahren zur Temperaturführung einer Dampf-Dealkylierung**

(30) Priorität: 26.01.2007 DE 102007004075
(71) Anmelder: Linde Aktiengesellschaft, 80807 München (DE)
(72) Erfinder: Fritz, Helmut, 81373 München (DE); Göke, Volker, 82515 Wolfratshausen (DE)

(57) **Zusammenfassung**

Die Erfindung beschreibt ein Verfahren zur Temperaturführung einer Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer mit Katalysatormaterial gefüllten und von außen beheizten Vorrichtung in einer Anlage zur Erzeugung von Kohlenwasserstoffen aus kohlenwasserstoffhaltigem Einsatz. Die jeweilige Kohlenwasserstofffraktion wird mit dem Wasserdampf in der gasförmigen Phase gemischt und an dem festen Katalysator in der Vorrichtung vorbeigeführt. Die Eintrittstemperatur der Einsatzstoffe liegt über der Austrittstemperatur der Reaktionsprodukte.

## Beschreibung

Die Erfindung betrifft ein Verfahren zur Temperaturführung einer Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer mit Katalysatormaterial gefüllten und von außen beheizten Vorrichtung in einer Anlage zur Erzeugung von Kohlenwasserstoffen aus kohlenwasserstoffhaltigem Einsatz.

In einer Anlage zur Erzeugung von Kohlenwasserstoffen aus kohlenwasserstoffhaltigem Einsatz entsteht ein Gemisch aus Kohlenwasserstoffen mit einer unterschiedlichen Anzahl von Kohlenstoffatomen und unterschiedlichen molekularen Konfigurationen. Dieses Gemisch wird in der Anlage zur Erzeugung von Kohlenwasserstoffen aus kohlenwasserstoffhaltigem Einsatz in die einzelnen Kohlenwasserstoffprodukte aufgetrennt.

Abhängig von der jeweiligen Trennsequenz und der Art der Anlage zur Erzeugung von Kohlenwasserstoffen aus kohlenwasserstoffhaltigem Einsatz entstehen eine Kohlenwasserstofffraktion mit mindestens sechs Kohlenstoffatomen (C₆₊-Fraktion) und/oder eine Kohlenwasserstofffraktion mit mindestens sieben Kohlenstoffatomen (C₇₊-Fraktion). Diese Fraktionen enthalten als wirtschaftlich verwertbares Produkt Aromate, welche als Ausgangsstoff für die Synthese zahlreicher Kunststoffe und zur Erhöhung der Klopffestigkeit von Benzin Verwendung finden.

Um an die wirtschaftlich verwertbaren Produkte der C₆₊-Fraktion, vor allem Benzol, zu gelangen und die Ausbeute möglichst maximal zu gestalten, wird nach dem Stand der Technik folgendes Verfahren angewendet. Die C₆₊-Fraktion wird in eine Fraktion überwiegend bestehend aus Kohlenwasserstoffen mit sechs Kohlenstoffatomen (C₆-Fraktion) und eine C₇₊-Fraktion getrennt. Aus der C₆-Fraktion kann direkt das wirtschaftlich verwertbare Produkt Benzol abgetrennt werden. Aus der C₇₊-Fraktion werden mittels einer Flüssig-Flüssig Extraktion die linearen Kohlenwasserstoffe abgetrennt und als so genanntes Raffinat weiterverarbeitet. Die von den linearen Kohlenwasserstoffen befreite C₇₊-Fraktion enthält nunmehr hauptsächlich Aromate mit sieben bis acht Kohlenstoffatomen und wird in eine Fraktion überwiegend bestehend aus Kohlenwasserstoffen mit sieben Kohlenstoffatomen (hauptsächlich Toluol) und in eine Fraktion überwiegend bestehend aus Kohlenwasserstoffen mit acht Kohlenstoffatomen (hauptsächlich Xylol) getrennt. Die Fraktion überwiegend bestehend aus Kohlenwasserstoffen mit acht Kohlenstoffatomen kann als Einsatzstoff in einem Verfahren zur para-Xylol Gewinnung dienen. Die Fraktion überwiegend bestehend aus Kohlenwasserstoffen mit sieben Kohlenstoffatomen wird als Einsatz in ein Verfahren zur Hydro-Dealkylierung geführt wird.

Ein derartiges Verfahren zur Hydro-Dealkylierung ist zum Beispiel in WO2005071045 beschrieben. Die Kohlenwasserstoffe werden in Anwesenheit eines Katalysators bei einer Temperatur von 400°C bis 650°C und einem Druck zwischen 20 bar und 40 bar mit Wasserstoff kontaktiert, wobei der Wasserstoff in einem molaren Überschuss vom drei- bis sechsfachen der Kohlenwasserstoffe vorliegt. Unter diesen Bedingungen werden die Alkylgruppen von den jeweiligen alkylierten Aromaten (wie zum Beispiel Toluol) abgespalten; so dass sich Benzol und die jeweiligen Alkane (zum Beispiel Methan) bilden.

Der Verbrauch von Wasserstoff bei der Hydro-Dealkylierung der Kohlenwasserstoffe wirkt sich negativ auf die Wirtschaftlichkeit dieses Verfahrens nach dem Stand der Technik zur Benzolgewinnung aus.

In einem alternativen Verfahren werden die C₆₊-Fraktion und/oder die C₇₊-Fraktion einer Dampf-Dealkylierung unterzogen (siehe zum Beispiel eingereichte Patentschriften DE102006058528.3 oder DE102006058529.1). Bei einer Dampf-Dealkylierung der C₆₊-Fraktion und/oder C₇₊-Fraktion entstehen hauptsächlich die beiden verwertbaren Produktstoffe Benzol und Wasserstoff neben Reaktionsprodukten wie Kohlenmonoxid und Kohlendioxid. Als Einsatzstoff der Dampf-Dealkylierung wird somit lediglich kostengünstiger Wasserdampf bei gleichzeitiger Produktion des gewünschten Wertproduktes Benzol und des wertvollen Nebenproduktes Wasserdampf benötigt.

In diesem Verfahren nach dem Stand der Technik reagieren die Kohlenwasserstoffe aus der jeweiligen Kohlenwasserstofffraktion mit Wasserdampf in der Gasphase an einem festen Katalysator. Dabei werden die Kohlenwasserstoffe und der Wasserdampf in die Gasphase überführt, gemischt und anschließend in eine Vorrichtung, zum Beispiel Rohre, geführt. Das Innere der Vorrichtung, zum Beispiel das Rohrinnere, ist mit einem festen Katalysatormaterial gefüllt, an dem die gasförmigen Reaktanten vorbeigeführt werden. Die für die Dealkylierung nötige Reaktionswärme wird der Vorrichtung von außen zugeführt, zum Beispiel durch Verbrennung der gasförmigen Reaktionsnebenprodukte Kohlenmonoxid und Methan mit Luft. Nach dem Stand der Technik entspricht die Temperatur der Einsatzstoffe der Austrittstemperatur der Reaktionsprodukte und damit der Reaktionstemperatur in der Vorrichtung. Die Eintritts- bzw. Austrittstemperatur nach dem Stand der Technik liegt im Bereich von 400 °C bis 600° C.

Bei dem beschriebene Verfahren nach dem Stand der Technik zur Dampf-Dealkylierung bildet sich im Eingangsbereich der von außen beheizten Vorrichtung eine Temperatursenke aus (als Eingansbereich der Vorrichtung wird im Folgenden der mit Katalysatormaterial befüllte Teil der Vorrichtung bezeichnet, welcher am Anfang von den gasförmigen Reaktionsprodukten durchströmt wird). Im Eingangsbereich der Vorrichtung beginnt die Dealkylierungsreaktion der Kohlenwasserstoffe mit dem Wasserdampf unter Wärmeverbrauch. Dadurch sinkt hier die Temperatur im Katalysatormaterial deutlich unter die Eintrittstemperatur der Einsatzstoffe auf ein Temperaturniveau, auf dem die Dealkylierungsreaktion nicht so effektiv ist. Um die Temperatur wieder auf das Niveau der Reaktionstemperatur bzw. Austrittstemperatur zu heben beziehungsweise auf dem Niveau zu halten und somit eine effektive Dealkylierungsreaktion zu erreichen, muss ungünstig lokal überproportional viel Wärme zugeführt werden. Eine derartige lokal höhere Wärmezufuhr erfordert einen höheren apparativen Aufwand und wirkt sich ungünstig auf die gesamte der Vorrichtung zuzuführende Wärmemenge aus. Alternativ wird im Stand der Technik auf eine lokal höhere Wärmezufuhr verzichtet. Dies hat zur Folge, dass die Einsatzstoffe längere Zeit auf einem Temperaturniveau unter der optimalen Reaktionstemperatur verbleiben und somit die Effektivität der Dealkylierungsreaktion für eine längere Zeit verringert ist. Um den gleichen Umsatz der Einsatzstoffe zu erzielen, muss bei diesem Verfahren nach dem Stand der Technik die Größe der Vorrichtung, zum Beispiel die Rohrlänge, erhöht werden. Eine derartige Vergrößerung der Vorrichtung führt zu einer Erhöhung des mit teurem Katalysatormaterial zu befüllenden Volumens und wirkt sich somit negative auf die Kosten der Anlage aus.

Der vorliegenden Erfindung liegt daher die Aufgabe zugrunde, ein Verfahren der eingangs erwähnten Art derart auszugestalten, dass eine erhöhte lokale Wärmezufuhr in die Vorrichtung vermieden wird und dabei auf eine Vergrößerung der Vorrichtung verzichtet werden kann.

Die gestellte Aufgabe wird erfindungsgemäß dadurch gelöst, dass die Eintrittstemperatur der Einsatzstoffe der Dampf-Dealkylierung über der Austrittstemperatur des Produktgemisches der Dampf-Dealkylierung liegt.

Durch die Erhöhung der Eintrittstemperatur der Einsatzstoffe der Dampf-Dealkylierung über die Austrittstemperatur des Produktgemisches wird eine homogenere Temperaturverteilung über die gesamte Vorrichtung erreicht und somit die Gesamtmenge der zuzuführenden Wärme minimiert. Werden die Einsatzstoffe mit einer erfindungsgemäß höheren Eintrittstemperatur als die Austrittstemperatur, d.h. einer Eintrittstemperatur die über der Reaktionstemperatur liegt, in die Vorrichtung geführt, vermindert sich die Temperatur der gasförmigen Einsatzstoffe und im Eingangsbereich der Vorrichtung bereits gebildeten Reaktionsprodukte lediglich auf die gewünschte Reaktionstemperatur. Der Vorrichtung muss dann im weiteren Verlauf nur soviel Wärme zugeführt werden, wie nötig ist, um die Reaktionstemperatur aufrecht zu erhalten. Der dafür erforderliche Wärmebedarf in der Vorrichtung ist durch die Anwendung des erfindungsgemäßen Verfahrens wesentlich gleichmäßiger verteilt und zugleich wird die Gesamtmenge der der Vorrichtung zuzuführenden Wärme minimiert. Zusätzlich sinkt an keiner Stelle der Vorrichtung die Temperatur unter die optimale Reaktionstemperatur, so dass an keiner Stelle der Vorrichtung die Umsatzrate der Dealkylierung durch ein unzureichendes Temperaturniveau negativ beeinflusst wird. Dadurch kann auf eine Vergrößerung der Vorrichtung verzichtet und eine kompakte Bauweise erreicht werden.

Vorteilhafterweise liegt die Eintrittstemperatur der Einsatzstoffe der Dampf-Dealkylierung über der mittleren Temperatur im Katalysatormaterial. Durch die Anwendung des erfindungsgemäßen Verfahrens ist die über den Querschnitt der Vorrichtung senkrecht zur Fließrichtung der Reaktanten gemittelte Temperatur über die gesamte Vorrichtung mit Ausnahme des Eingangsbereiches nahezu konstant und gleich der Austrittstemperatur des Produktgemisches der Dampf-Dealkylierung.

Zweckmäßigerweise liegt die Eintrittstemperatur der Einsatzstoffe der Dampf-Dealkylierung unter 650 °C, bevorzugt unter 600 °C, besonders bevorzugt unter 550 °C und die Austrittstemperatur des Produktgemisches im Bereich von 400 °C bis 500°C, bevorzugt von 420 °C bis 480 °C, besonders bevorzugt von 430 °C bis 470 °C.

Als ebenfalls vorteilhaft erweist sich die Erwärmung der Einsatzstoffe der Dampf-Dealkylierung auf die Eintrittstemperatur durch die Abwärme der Beheizung der mit Katalysatormaterial gefüllten und beheizten Vorrichtung. In einer besonders bevorzugten Ausgestaltung der Erfindung wird die für die Dampf-Dealkylierung notwendige Wärme durch eine Verbrennung erzeugt und die Einsatzstoffe der Dampf-Dealkylierung durch Wärmeaustausch mit den Abgasen der Verbrennung erwärmt. Dadurch wird die Abwärme der Beheizung sinnvoll genutzt und für die Vorwärmung der Einsatzstoffe der Dampf-Dealkylierung keinerlei zusätzlich Wärme notwendig, wodurch wiederum die Gesamtmenge der dem Prozess zuzuführenden Wärme minimiert wird.

In einer bevorzugten Ausgestaltung der Erfindung werden die jeweilige Kohlenwasserstofffraktion und der Wasserdampf als Gemisch in der gasförmigen Phase direkt vor dem Eintritt in die Vorrichtung auf die Eintrittstemperatur erwärmt.

Mit der vorliegenden Erfindung gelingt es insbesondere auf eine aufwändige erhöhte lokale Wärmezufuhr in die Vorrichtung zu verzichten und gleichzeitig eine kompakte Bauweise zu ermöglichen.

## Patentansprüche

1. Verfahren zur Temperaturführung einer Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer mit Katalysatormaterial gefüllten und von außen beheizten Vorrichtung in einer Anlage zur Erzeugung von Kohlenwasserstoffen aus kohlenwasserstoffhaltigem Einsatz, **dadurch gekennzeichnet, dass** die Eintrittstemperatur der Einsatzstoffe der Dampf-Dealkylierung über der Austrittstemperatur des Produktgemisches der Dampf-Dealkylierung liegt.

2. Verfahren nach Anspruch 1, **dadurch gekennzeichnet, dass** die Eintrittstemperatur der Einsatzstoffe der Dampf-Dealkylierung über der Temperatur liegt, die sich als mittlere Temperatur im Katalysatormaterial ergibt.

3. Verfahren nach Anspruch 1 oder 2, **dadurch gekennzeichnet, dass** die Eintrittstemperatur der Einsatzstoffe der Dampf-Dealkylierung unter 650 °C, bevorzugt unter 600 °C, besonders bevorzugt unter 550 °C, liegt.

4. Verfahren nach einem der Ansprüche 1 bis 3, **dadurch gekennzeichnet, dass** die Austrittstemperatur des Produktgemisches im Bereich von 400 °C bis 500°C, bevorzugt von 420°C bis 480 °C, besonders bevorzugt von 430 °C bis 470 °C, liegt.

5. Verfahren nach einem der Ansprüche 1 bis 4, **dadurch gekennzeichnet, dass** die Einsatzstoffe der Dampf-Dealkylierung auf die Eintrittstemperatur durch die Abwärme der Beheizung der mit Katalysatormaterial gefüllten und beheizten Vorrichtung erwärmt werden.

6. Verfahren nach einem der Ansprüche 1 bis 5, **dadurch gekennzeichnet, dass** die für die Dampf-Dealkylierung notwendige Wärme durch eine Verbrennung erzeugt wird und die Einsatzstoffe der Dampf-Dealkylierung durch Wärmeaustausch mit den Abgasen der Verbrennung erwärmt werden.

7. Verfahren nach einem der Ansprüche 1 bis 6, **dadurch gekennzeichnet, dass** die jeweilige Kohlenwasserstofffraktion und der Wasserdampf als Gemisch in der gasförmigen Phase direkt vor dem Eintritt in die Vorrichtung auf die Eintrittstemperatur erwärmt werden.
